# EUROPEAN PATENT SPECIFICATION

(11) **EP 0 435 518 B1**
(45) Date of publication and mention of the grant of the patent: **15.02.1995**
(21) Application number: 90313604.2
(22) Date of filing: 13.12.1990
(51) Int. Cl.: A61M 25/00

(54) **A flexible, kink-resistant catheter**
Flexibler knickbeständiger Katheter
Cathéter à l'épreuve de flambage flexible

(30) Priority: 29.12.1989 US 458610
(43) Date of publication of application: 03.07.1991
(73) Proprietor: MED INSTITUTE, INC., West Lafayette Indiana 47906 (US)
(72) Inventor: Fearnot, Neal Edward, West Lafayette, Indiana 47906 (US); Sisken, Richard Brian, West Lafayette, Indiana 47906 (US)
(74) Representative: Johnston, Kenneth Graham

(56) References cited:
- FR-A- 2 204 430
- FR-A- 2 505 191
- US-A- 3 757 768
- US-A- 3 841 308
- US-A- 4 425 919

## Description

This invention relates to catheters.

Catheters have various uses, one being to provide regional anaesthesia which minimizes the total dosage of inhalation or intravenous anaesthetic drugs required during operations, and hastens awakening, and permits early ambulation afterwards. When administered at the conclusion of surgery, regional anaesthesia produces post-operative analgesia with reduced risk of respiratory depression. Furthermore, certain types of pain are difficult to treat with systemic narcotics.

Caudal epidural anaesthesia is notable for its simplicity, safety, and effectiveness and is one of the most frequently used regional anaesthetic techniques for operations below the diaphragm in children.

Presently available catheters (having outside diameters of 0.107 or 0.089 cms (19 or 20 gauge)) are extraordinarily cumbersome to use in children.

The smallest presently available epidural catheter has an outside diameter of approximately 0.089cms (0.035"). It is constructed of a tightly wound helix within a plastic tube whose inner surface conforms to the outer surface of the spiral helix, the thickness of the polymer coating thus varying according to the contour of the outside surface of the helix. When such a catheter is flexed or bent the polymer coating is subjected to forces that are more than sufficient to rupture or tear the polymer coating. The coating is unable to slide or move longitudinally over the outside surface of the helix, and easily ruptures. The catheter may have fluid pressure limitations as well.

The windings at the distal end of this catheter are uncoated and such windings when expanded are susceptible to the ingrowth of tissue, particularly with long-term placement. Removal of the catheter will thus cause trauma.

It is obviously desirable to reduce the outside diameter of a catheter to as small a value as is practical. However, reducing the dimensions of existing catheters introduces significant problems, one of them being that one must maintain a minimum fluid volume delivery rate, which would be limited by the inside diameter of the catheter, the thickness of the catheter wall, and the pressure at which the fluid is delivered to the catheter. Simply reducing the outside diameter of the catheter, while maintaining the inside diameter, decreases the thickness of the catheter wall and introduces a number of other concerns, such as kinking when flexed or bent and the maximum pressure at which the fluid may be delivered without rupturing the catheter wall. As the thickness of the catheter wall decreases, susceptibility of the catheter to kinking increases. At a minimum, kinking of the catheter wall reduces the fluid volume delivery rate and, in many cases, causes a fluid stoppage and a rupture of the catheter wall with an accompanying loss of fluid. Furthermore, the reduced wall thickness must be capable of withstanding the delivery pressure without rupture.

Flexibility of the catheter is increased with thinner wall thicknesses. However, the susceptibility of the catheter to kinking when flexed or bent also increases along with the probability of rupture due to kinking or high fluid pressures.

Minimum fluid volume delivery rates may be maintained with an increased delivery pressure when the inside and outside diameters of the catheter are decreased. However, an increase in delivery pressure must not cause injury to the tissue in the vicinity of a catheter.

A number of small diameter catheters less than 0.084cms (0.033") have been attempted with one or more layers of polymer plastic material. Such a catheter is known from US-A-3 757 768 on which the preamble of claim 1 is based. However, the interrelationship between flexibility, kink-resistance, and fluid delivery pressure has prevented the introduction of an all plastic material catheter that is less than 0.084cms (0.033") in diameter and that is flexible, kink-resistant, and capable of delivering a volume of fluid at a rate which is acceptable by the medical community.

According to the present invention there is provided a catheter as defined in claim 1.

The coil is either friction-fitted within the sheath or has a circumference that is in contact with the inside surface of the tubular sheath to improve the kink-restance of the flexible sheath.

The structure provides a flexible, kink-resistant catheter with an outside diameter providing acceptable delivery rates of fluid volume. This constitutes a significant departure in the art.

The high tensile strength of the tubular material advantageously provides for the delivery of fluid at more than acceptable volume levels. The kink-resistance of the catheter wall is maintained at an extremely high level with the friction-fitted elongated spiral coil positioned within the passageway of the tubular sheath. The spiral coil distributes the radial and longitudinal forces over the high tensile strength tube without rupturing the tube when the catheter is flexed or bent. When flexed or bent, the effected coils move within the passageway of the tube to distribute the forces along the surface of the bent tube. The combination of the high tensile and flexural strength tubular sheath material and the spiral coil positioned within the passageway thereof allows the catheter to be virtually tied in a knot and still provide more than adequate levels of fluid volume delivery without rupturing or kinking the catheter.

The coil preferably extends substantially along the entire length of the sheath, and can have different spacings between adjacent turns to provide variable flexibilities along the catheter. In the preferred embodiment, there is provided one section with the turns closely coupled, namely with the turns touching or closely spaced with spacings of between about 1 tenth and 1 thousandth of an inch, or 0 to 5% of coil wire diameter, and a distal or other section with the turns loosely spaced with spacings of between about 5 tenths and 2 thousandths of an inch, or 2.5 to 10% of coil wire diameter. In these arrangements the turns are said to be variably spaced.

To further increase the flexibility of the distal end of the catheter, the turns of the spiral coil are relaxed so as to provide a spacing therebetween which is in contrast to the tightly coupled turns throughout the remaining portion of the catheter.
FIG.1 depicts a flexible, kink-resistant catheter of the present invention;
FIG.2 depicts a partial cross-sectional view of the distal end of the catheter of FIG.1;
FIG.3 depicts a second embodiment of the catheter of FIG.2 with an alternative safety wire; and
FIG.4 depicts an alternative embodiment of the catheter of FIG.1 with a tubular sheath positioned within the passageway of the wire coil.

### Detailed description

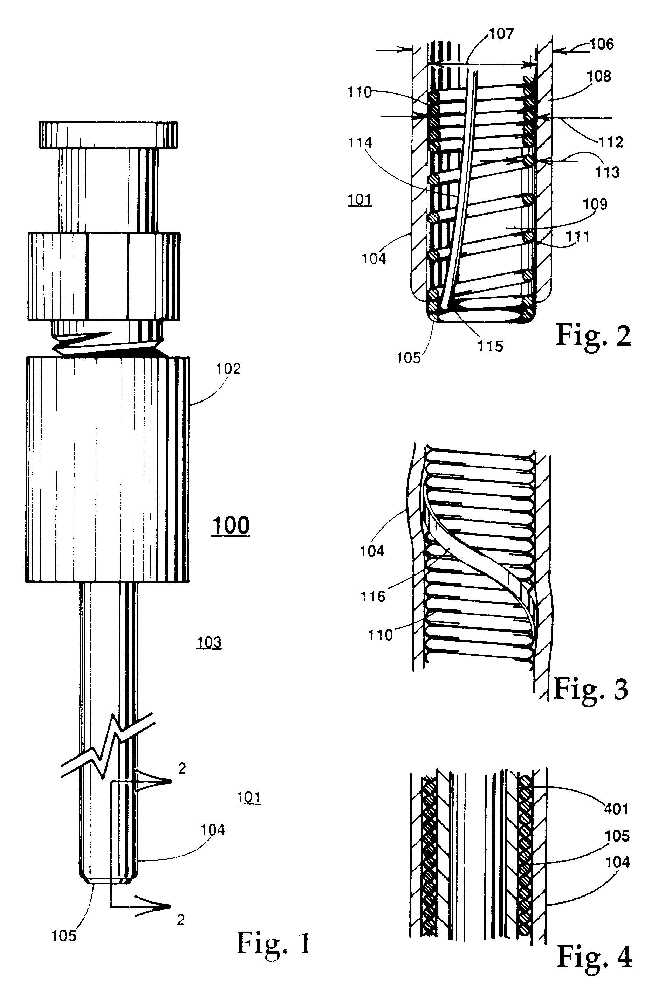

Depicted in FIG.1 is an illustrative epidural catheter 100 having a flexible, kink-resistant segment 101 which is passable through an aperture having a diameter less than 0.084cms (0.033"). A catheter segment with an outside diameter as small as 0.025cms (0.010") is available for insertion through a Tuohy or Crawford thin-wall needle with an outside diameter as small as 0.0308 cms (25-gauge). After the needle is inserted in the spine, the epidural catheter is inserted through the hollow passageway of the needle into the epidural or caudal space. When the catheter is in place, the needle is removed over the entire length of the catheter, and a well-known and commercially available medical grade connector 102, such as a Tuohy-Borst connector, which is available from Cook, Inc., is attached to the proximal end 103 of the catheter.

The flexible, kink-resistant elongated segment comprises a tubular sheath or tube 104 having a longitudinal passageway in which an elongated wire coil 105 is friction-fitted. The combination of the tubular sheath and the friction-fitted wire coil therein provides the catheter with a thin-wall structure which is both flexible and kink-resistant.

A partial cross-sectional view of flexible elongated segment 101 of the catheter is depicted in FIG.2. Tubular sheath 104 has an outside diameter 106 in a range of 0.0254 to 0.08cms (0.010" to 0.032"). The inside diameter 107 of the sheath is in a range of 0.02 to 0.066cms (0.008" to 0.026"). The thickness of tubular sheath wall 108 is in range of 0.00254 to 0.00762cms (0.001" to 0.003"). The tubular sheath comprises a material having a high tensile or flexural strength to resist tearing, rupturing, or bursting when either pulled, flexed or bent or when subjected to high pressure fluids therein. The tubular sheath comprises a plastic tube of polyimide material which is available from Micro ML Tubing Sales. This polyimide material tube comprises a flexible, non-flammable, radiation resistant and non-corrosive material. In addition, the polyimide material tubing exhibits a nominal tensile strength of 138.0 MPa (20,000 pounds per square inch) with an even higher flexural strength. This cross-linked polymer allows for a tubular sheath with an extremely small wall thickness for flexibility, but also has an extremely high tensile, as well as flexural, strength to resist rupturing or tearing when flexed or bent. The high tensile and flexural strength also provides for delivering fluids at extremely high pressure levels. Other materials such as polyamide and fluoropolymers having a tensile strength greater than 20.7 MPa (3000 pounds per square inch) and preferably 69.0 MPa (10,000 pounds per square inch) are also acceptable. A variety of commercially-available TEFLON material tubes are also acceptable for low pressure or less kink-resistant applications. Various other copolymer plastic materials may also be acceptable; however, these plastic materials must be capable of being formed into a tubular sheath with the afore-mentioned dimensions. Such extremely small diameter and wall thickness tubing made of these polymer plastic materials have been extremely diificult to extrude or fabricate with these dimensions.

Positioned within longitudinal passageway 109 of tubular sheath 104 is coil 105. Coil 105 comprises an elongated, spirally wound helical wire coil of a hardened stainless steel material. Coil 105 may also be made of other suitable materials such as other metals or alloys, carbon filaments, hard plastic fibers, or combinations thereof.

Coil 105 includes a plurality of closely-coupled turns 110 from the proximal end to almost the distal end of the catheter. Turns 110 of the coil are closely spaced for example to be in physical contact with each other with very little, if any, space between the individual windings or turns. At the distal end of coil 105 is a plurality of loosely coupled turns 111. These loosely coupled turns are formed from the tightly coupled turns by stretching or relaxing the distal end of the coil. The spacing between the turns of 111 provides further flexibility to the distal end of the catheter for atraumatic insertion into biological tissue.

The outside diameter of coil 105 is in a range of 0.02 to 0.066cms (0.008" to 0.026"). The cross-sectional diameter of the individual spiral members or turns of coil 105 are within a range of 0.00254 to 0.00762cms (0.001" to 0.003"). Coil 105 is positioned within passageway 109 of the sheath in one of several different ways. By way of a first example, the opposite ends of the coil are turned in opposing directions to cause the outside diameter of the coil to shrink. When the outside diameter of the coil is reduced, the tubular sheath is positioned thereover. The opposite ends of the coil are then released to permit the outside diameter of the turns to expand and form an expansion or friction fit against the inside surface of the tube. This fabrication method is preferred; however, all that is required is that the circumference of the spiral member is in contact with the inside surface of the tubular sheath. This close friction-fit contact between the tubular sheath and wire coil forms the thin-wall structure of the catheter which is extremely flexible and is resistant to rupture or tearing when subjected to bending or flexing.

Experimental results have indicated that when the ratio of the outside coil diameter to the spiral member diameter is maintained in a range of 4 to 10 with a tubular sheath thickness of 0.00254 to 0.00762cms (0.001"to 0.003"), the combined catheter wall structure with a polyimide sheath material does not rupture or kink. When the ratio is below 4, even the high tensile strength polyimide material could rupture or tear. When the ratio exceeds 10, the polyimide material tubular sheath could kink with occlusion of the passageway of the sheath.

Coil 105 such as a hardened stainless steel wire coil with the above cross-sectional dimensions and outside diameters are available from Cook, Inc., as well as other suppliers.

A safety wire 114 extends throughout the entire length of the passageway of the coil and tubular sheath. As shown, the distal ends of the safety wire and coil are attached using silver solder 115. The windings are also shaped to provide a rounded surface for atraumatic insertion into tissue. The distal end of the tubular sheath may also be rounded for further ease of insertion. The proximal ends of the safety wire and wire coil are also similarly attached. The safety wire prevents the stretching of the wire coil for whatever reason.

Depicted in FIG.3 is an alternative embodiment of a safety wire 116 spirally wound around the outside of the coil and the inside of the tubular sheath.

Depicted in FIG.4 is a partial cross-sectional view of flexible segment 101 with an inner tube 401 positioned within the passageway of coil 105. As a result, the wall thickness of outside tubular sheath 104 may be reduced. This alternative embodiment provides for a very smooth surface for which fluids without an anticoagulant may be delivered to biological tissue. This is particularly advantageous in those cases of long term insertion of a catheter. In addition, the flow of fluid is laminar rather than turbulent which provides for greater fluid volume delivery rates. Furthermore, the likelihood of particulates in any fluid coagulating around the windings of coil 105 are eliminated or reduced.

It is to be understood that the above-described flexible, kink-resistant catheter is merely an illustrative embodiment describing the principles of this invention and that other catheters may be devised by those skilled in the art. In particular, the tubular sheath may be formed of other materials not specifically mentioned herein, but have a minimal tensile or flexural strength for providing the combined wall structure. In addition, the spiral member of the wire coil may be formed from other materials not specifically mentioned herein. The sheath and coil materials may also be preshaped to improve access to any one of a number of curved body passageways. One or both ends of the catheter may be curved, tapered, or shaped to form totally implantable devices such as stents and the like. Slits or ports in the sheath may also be formed to provide side delivery and even greater dispersion of the fluids injected from the distal end of the catheter as described in the parent application. The passageway may further be utilized to insert other medical instruments such as a stylet, another catheter, or a wire guide.

## Claims

1. A catheter for insertion into biological tissue, said catheter comprising a tubular sheath (104) having a longitudinal passageway therein capable of transporting a fluid to the tissue, and being able to maintain fluid flow when said sheath is flexed; and an elongated coil (105) within and contacting said longitudinal passageway of said tubular sheath, characterised in that the outer surface of the coil and the inner surface of the sheath are friction fitted in order to permit frictional movement therebetween.

2. The catheter of claim 1, characterised in that the sheath has a nominal tensile strength greater than 20.7 MPa (3,000 pounds per square inch), or at least 138 MPa (20,000 pounds per square inch).

3. The catheter of claim 1, characterised in that said tubular sheath has an outside diameter less than 0.084cms (0.033"), and/or an inside dfiameter in a range of 0.02 cms to 0.066cms (0.008" to 0.026").

4. The catheter of claim 1, characterised in that said tubular sheath comprises a plastic material, or polyimide.

5. The catheter of claim 4, characterised in that the tubular sheath and the coil have a combined thickness in a range of 0.005cms to 0.015cms (0.002" to 0.006"), and/or the catheter has an outside diameter in a range of 0.0254cms to 0.08cms (0.010" to 0.032"), and/or said coil includes a spiral member having a cross-sectional dimension in a range of 0.00254cms to 0.00762cms (0.001" to 0.003").

6. The catheter of any one preceding claim, characterised in that said spiral member comprises a stainless steel wire.

7. The catheter of claim 1, characterised in that said coil has a passageway therein, and in that said catheter further comprises a second tube positioned within the passageway of said coil.

8. The catheter of any one preceding claim, further characterised by a strand either spirally positioned around a length of said elongated coil and attached about the opposite ends thereof, or positioned through a passageway of said coil and attached about the opposite ends thereof.

9. The catheter of any one preceding claim, characterised in that the turns of said coil are variably spaced.

10. The catheter of claim 9, characterised in that the coil includes a first plurality of turns and a second plurality of turns positioned about a distal end thereof, the second plurality being more loosely coupled than said first plurality of turns.

11. The catheter of claim 3, characterised in that the sheath has a wall thickness in a range of 0.00254 to 0.00762cms (0.001" to 0.003"), and/or the coil has an outside diameter in a range of 0.02 to 0.066cms (0.008" to 0.026").

12. The catheter of claim 11, characterised in that the ratio of the outside coil diameter to said member diameter is in a range of 4 to 10.

13. The catheter of claim 7, characterised in that the second tube has a wall thickness in a range of 0.00254 to 0.00762cms (0.001" to 0.003").

## Patentansprüche

1. Katheter zum Einschieben in biologisches Gewebe, bestehend aus einem rohrförmigen Mantel (104) mit einem Längsdurchgang darin, der zum Transport einer Flüssigkeit zum Gewebe befähigt ist und bei Biegung jenes Mantels die Flüssigkeitsströmung aufrechterhalten kann, und aus einer länglichen Spule (105) in und in Berührung mit diesem Längsdurchgang jenes rohrförmigen Mantels, dadurch gekennzeichnet, daß die Außenfläche der Spule und die Innenfläche des Mantels auf Reibung eingepaßt sind, um eine reibende Bewegung dazwischen zuzulassen.

2. Katheter nach Anspruch 1, dadurch gekennzeichnet, daß der Mantel eine höhere Nennzugfestigkeit als 20,7 MPa (3,000 Pfund pro Quadratzoll) oder mindestens 138 MPa (20,000 Pfund pro Quadratzoll) aufweist.

3. Katheter nach Anspruch 1, dadurch gekennzeichnet, daß dieser rohrförmige Mantel einen kleineren Außendurchmesser als 0,084 cm (0,033") und/oder einen Innendurchmesser im Bereich von 0,02 cm bis 0,066 cm (0,008" bis 0,026") aufweist.

4. Katheter nach Anspruch 1, dadurch gekennzeichnet, daß dieser rohrförmige Mantel aus einem Kunststoff bzw. Polyimid besteht.

5. Katheter nach Anspruch 4, dadurch gekennzeichnet, daß der rohrförmige Mantel und die Spule eine kombinierte Dicke im Bereich von 0,005 cm bis 0,015 cm (0,002" bis 0,006") besitzen und/oder der Katheter einen Außendurchmesser im Bereich von 0,0254 cm bis 0,08 cm (0,010" bis 0,032") aufweist und/oder diese Spule ein Spiralglied mit einer Querschnittsabmessung im Bereich von 0,00254 cm bis 0,00762 cm (0,001" bis 0,003") einschließt.

6. Katheter nach einem der vorhergehenden Ansprüche, dadurch gekennzeichnet, daß dieses Spiralglied einen Edelstahldraht enthält.

7. Katheter nach Anspruch 1, dadurch gekennzeichnet, daß diese Spule in sich einen Durchgang aufweist und daß dieser Katheter ferner ein zweites, im Durchgang dieser Spule angeordnetes Rohr enthält.

8. Katheter nach einem der vorhergehenden Ansprüche, ferner gekennzeichnet durch einen Strang, der entweder um eine Länge jener länglichen Spule herum spiralförmig angeordnet und um deren entgegengesetzte Enden herum befestigt ist, oder der durch einen Durchgang jener Spule hindurch angeordnet und um deren entgegengesetzte Enden herum befestigt ist.

9. Katheter nach einem der vorhergehenden Ansprüche, dadurch gekennzeichnet, daß die Windungen dieser Spule variabel beabstandet sind.

10. Katheter nach Anspruch 9, dadurch gekennzeichnet, daß die Spule eine erste Mehrzahl Windungen sowie eine zweite, etwa an deren distalem Ende angeordnete Mehrzahl Windungen aufweist, wobei die zweite Mehrzahl Windungen loser als die erste Mehrzahl angekoppelt ist.

11. Katheter nach Anspruch 3, dadurch gekennzeichnet, daß der Mantel eine Wandstärke im Bereich von 0,00254 bis 0,00762 cm (0,001" bis 0,003") und/oder die Spule einen Außendurchmesser im Bereich von 0,02 bis 0,066 cm (0,008" bis 0,026") aufweist.

12. Katheter nach Anspruch 11, dadurch gekennzeichnet, daß das Verhältnis des Spulenaußendurchmessers zu jenem Glieddurchmesser im Bereich von 4 bis 10 liegt.

13. Katheter nach Anspruch 7, dadurch gekennzeichnet, daß das zweite Rohr eine Wandstärke im Bereich von 0,00254 bis 0,00762 cm (0,001" bis 0,003") aufweist.

## Revendications

1. Cathéter à insérer dans un tissu biologique, ledit cathéter comprenant une gaine tubulaire (104) munie d'un passage longitudinal capable de transporter un fluide vers le tissu, et capable de maintenir un écoulement de fluide lorsque ladite gaine est fléchie; et un serpentin allongé (105) à l'intérieur dudit passage longitudinal et en contact avec ce dernier, caractérisé en ce que la surface externe du serpentin et la surface interne de la gaine sont assemblés en frottement afin de permettre entre les deux un mouvement de frottement.

2. Cathéter selon la revendication 1, caractérisé en ce que la gaine possède une résistance nominale à la traction supérieure à 20,7 MPa (3 000 psi), ou d'au moins 138 MPa (20 000 psi).

3. Cathéter selon la revendication 1, caractérisé en ce que ladite gaine tubulaire possède un diamètre extérieur inférieur à 0,084 cm (0,033") et/ou un diamètre intérieur dans la gamme de 0,02 cm à 0,066 cm (0,008" à 0,026").

4. Cathéter selon la revendication 1, caractérisé en ce que ladite gaine tubulaire comprend une matière plastique ou un polyimide.

5. Cathéter selon la revendication 4, caractérisé en ce que la gaine tubulaire et le serpentin possèdent une épaisseur combinée dans la gamme de 0,005 cm à 0,015 cm (0,002" à 0,006"), et/ou le cathéter possède un diamètre extérieur dans la gamme de 0,0254 cm à 0,08 cm (0,010" à 0,032") et/ou ledit serpentin comprend un élément en spirale ayant un diamètre de section droite dans la gamme de 0,00254 cm à 0,00762 cm (0,001" à 0,003").

6. Cathéter selon l'une quelconque des revendications précédentes, caractérisé en ce que ledit élément en spirale comprend un fil d'acier inoxydable.

7. Cathéter selon la revendication 1, caractérisé en ce que ledit serpentin est muni d'un passage et en ce que ledit cathéter comprend aussi un second tube placé à l'intérieur du passage dudit serpentin.

8. Cathéter selon l'une quelconque des revendications précédentes, caractérisé en outre par un fil placé en spirale, entourant une longueur dudit serpentin allongé et fixé au voisinage de ses extrémités opposées, ou bien placé à l'intérieur du passage dudit serpentin et fixé au voisinage de ses extrémités opposées.

9. Cathéter selon l'une quelconque des revendications précédentes, caractérisé en ce que les spires dudit serpentin ont un espacement variable.

10. Cathéter selon la revendication 9, caractérisé en ce que le serpentin comporte une première pluralité de spires et une second pluralité de spires placées au voisinage de son extrémité distale, la seconde pluralité de spires étant moins serrée que ladite première pluralité de spires.

11. Cathéter selon la revendication 3, caractérisé en ce que la gaine a une épaisseur de paroi dans la gamme de 0,00254 cm à 0,00762 cm (0,001" à 0,003") et/ou le serpentin a un diamètre extérieur dans la gamme de 0,02 cm à 0,066 cm (0,008" à 0,026").

12. Cathéter selon la revendication 11, caractérisé en ce que le rapport entre le diamètre extérieur du serpentin et le diamètre dudit élément est dans la gamme de 4 à 10.

13. Cathéter selon la revendication 7, caractérisé en ce que le second tube a une épaisseur de paroi dans la gamme de 0,00254 cm à 0,00762 cm (0,001" à 0,003").
